# EUROPEAN PATENT SPECIFICATION

(11) **EP 4 236 766 B1**
(45) Date of publication and mention of the grant of the patent: **03.06.2026**
(21) Application number: 21798396.4
(22) Date of filing: 29.10.2021
(51) Int. Cl.: A61B 5/00, A61B 5/145, A61B 5/1455

(54) **ACTIVE MINIATURIZED SENSING SYSTEM**
AKTIVES MINIATURISIERTES ERFASSUNGSSYSTEM
SYSTÈME DE DÉTECTION MINIATURISÉ ACTIF

(30) Priority: 02.11.2020 US 202063108551 P; 03.11.2020 EP 20205438
(43) Date of publication of application: 06.09.2023
(73) Proprietor: Glucomat GmbH, 93309 Kelheim (DE)
(72) Inventor: REICHL, Mathias, 93326 Abensberg (DE)
(74) Representative: Weickmann & Weickmann PartmbB
(86) International application number: PCT/EP2021/080191
(87) International publication number: WO 2022/090503

(56) References cited:
- WO-A1-2018/122319
- WO-A1-2019/160272
- US-A- 6 025 597
- US-A1- 2015 366 490

## Description

The present invention relates to a non-invasive active sensing system for determining a physiological parameter in a bodily fluid of subject. Further, the present invention relates to a non-invasive method for determining a physiological parameter in a bodily fluid of a subject.

### Background

In the year 2016, about 415 million people suffered from diabetes. For 2040, an increase to more than 640 million people can be expected. Since people with diabetes are at risk for complications such as blindness, kidney diseases, heart diseases and stroke, there is a need to control the disease by closely monitoring blood glucose level.

Presently, determination of blood glucose is mainly based on invasive system and methods, wherein either a blood sample is taken and subsequently subjected to an *in vitro* test or a sensor is implanted for determining the glucose level *in vivo.* These invasive systems and methods are disadvantageous in that they are painful or inconvenient.

Thus, there is a need for developing a system and methods, which allow a reliable non-invasive determination of glucose and or physiological parameters.

Document WO 2019/160272 A1 discloses a non-invasive blood glucose measurement method comprising the steps of:
irradiating near-infrared rays, e.g. at a wavelength between 1.5 µm and 3 µm, on the skin to be measured to increase the temperature of the skin;
measuring a mid-infrared spectrum, e.g. from 8 µm to 14 µm, generated from the skin at elevated temperature;
measuring an increase temperature of the skin by the near-infrared rays; and calculating a blood glucose level based on the skin temperature measurement and the mid-infrared spectrum.

### Summary of the invention

The invention is defined by the appended claims.

According to the present invention, a simple, rapid and reliable determination of a physiological parameter is feasible using non-invasive systems and methods. These systems and methods involve irradiation of a body part of a subject, particular a human subject, with visual (VIS)/near-infrared (NIR) radiation in the range of about 400 nm to about 1500 nm or about 500 nm to about 1500 nm and detecting emitted IR radiation from the irradiated body part of said subject in the range of about 5 µm to about 12 µm. Surprisingly, the present inventor has found that irradiating a body part, e.g. a fingertip, an earlobe, a wrist or a forearm with short-wavelength radiation and detecting emitted long-wavelength radiation from the irradiated body part allows determination for physiological parameters such as glucose in a bodily fluid such as blood. Irradiation of a body part with VIS/NIR radiation according to the present invention causes energy absorption within an area of the irradiated body part. Energy absorption in this irradiated area, i.e., the absorption area, results in a local increase of tissue temperature within the irradiated body part, particularly within the absorption area, which again causes an increased emission of IR radiation from the irradiated body part, particularly from the absorption area, including an increased emission of IR radiation in the range of about 5 µm to about 12 µm. Further, by means of the local temperature increase in the irradiated absorption area, the IR radiation emitted from the absorption area shifts away from the corresponding IR absorption maxima of the molecules of the physiological parameter, e.g., from the glucose molecules. Thus, detection of emitted IR radiation from the irradiated body part is facilitated and substantially improved.

A first aspect of the disclosure relates to a non-invasive system for determining a physiological parameter, particularly glucose, in a bodily fluid of a subject comprising:
(a) a radiation source adapted for emitting visual (VIS)/near-infrared (NIR) radiation in the range of about 400 nm to about 1500 nm into a body part of said subject, wherein the body part is particularly selected from a fingertip, an earlobe, a wrist, a forearm, a palm and an upper arm,
(b) a sensing unit for detecting emitted IR radiation from the irradiated body part of said subject in the range of about 5 µm to about 12 µm, wherein said sensing unit is adapted for (i) detecting IR radiation having at least one wavelength or wavelength range where the intensity of the detected IR radiation is dependent from the concentration of the physiological parameter in the bodily fluid of said subject, and for (ii) detecting IR radiation having at least one wavelength or wavelength range where the intensity of the detected IR radiation is substantially independent from the concentration of the physiological parameter in the bodily fluid of said subject, and
(c) an analyzing unit for the qualitative and/or quantitative determination of the physiological parameter based on the IR radiation detected in the sensing unit (b).

A further aspect of the disclosure relates to a device comprising a non-invasive system for determining a physiological parameter in a bodily fluid of a subject, wherein the device comprises a casing, wherein the device includes:
(a) a radiation source adapted for emitting visual (VIS)/near-infrared (NIR) radiation in the range of about 400 nm to about 1500 nm into a body part of said subject, wherein the body part is particularly selected from a fingertip, a plurality of finger tips, and a palm, and wherein the radiation source is further adapted the irradiated body part absorbs electromagnetic energy resulting in a local increase of tissue temperature and in an increased emission of IR radiation in the wavelength range of about 5 µm to about 12 µm,
(b) a sensing unit for detecting emitted IR radiation from the previously irradiated body part of said subject in the range of about 5 µm to about 12 µm,
   wherein said sensing unit is adapted for (i) detecting IR radiation having at least one wavelength or wavelength range where the intensity of the detected IR radiation is dependent from the concentration of the physiological parameter in the bodily fluid of said subject,
   and for (ii) detecting IR radiation having at least one wavelength or wavelength range where the intensity of the detected IR radiation is substantially independent from the concentration of the physiological parameter in the bodily fluid of said subject, and
(c) an analyzing unit for the qualitative and/or quantitative determination of the physiological parameter based on the IR radiation detected in the sensing unit (b), and

wherein the casing comprises a first face comprising a screen, wherein the screen is at least partially made of a material, which is optically transparent for NIR/VIS radiation emitted by the radiation source (a) and for IR radiation detected by the sensing unit (b),
wherein the radiation source (a), the sensing unit (b) and the analyzing unit (c) are incorporated within the casing.

A further aspect of the disclosure relates to the use of the above system and device for non-invasively determining a physiological parameter in a bodily fluid of a subject, particularly wherein the physiological parameter is glucose, and the bodily fluid is blood.

A still further aspect of the disclosure relates to a method for non-invasively determining a physiological parameter, particularly glucose in a bodily fluid of a subject comprising the steps:
(a) irradiating a body part of said subject with visual (VIS)/near-infrared (NIR) radiation in the wavelength range of about 400 nm to about 1500 nm,
(b) detecting emitted IR radiation from the irradiated body part of said subject in the wavelength range of about 5 µm to about 12 µm, comprising separately (i) detecting IR radiation having at least one wavelength or wavelength range where the intensity of the detected IR radiation is dependent from the concentration of the physiological parameter in the bodily fluid of said subject, and (ii) detecting IR radiation having at least one wavelength or wavelength range where the intensity of the detected IR radiation is substantially independent from the concentration of the physiological parameter in the bodily fluid of said subject, and
(c) analyzing the detected IR radiation for the qualitative and/or quantitative determination of the physiological parameter.

Yet a further aspect of the disclosure relates to a method for non-invasively determining a physiological parameter in a bodily fluid of a subject comprising the steps:
(a) irradiating a body part of said subject with visual (VIS)/near-infrared (NIR) radiation in the wavelength range of about 400 nm to about 1500 nm, or about 500 nm to about 1500 nm, wherein the irradiated body part absorbs electromagnetic energy resulting in a local increase of tissue temperature and in an increased emission of IR radiation in the wavelength range of about 5 µm to about 12 µm,
(b) detecting emitted IR radiation from the previously irradiated body part of said subject in the wavelength range of about 5 µm to about 12 µm, comprising separately (i) detecting IR radiation having at least one wavelength or wavelength range where the intensity of the detected IR radiation is dependent from the concentration of the physiological parameter in the bodily fluid of said subject, and
   (ii) detecting IR radiation having at least one wavelength or wavelength range where the intensity of the detected IR radiation is substantially independent from the concentration of the physiological parameter in the bodily fluid of said subject, and
(c) analyzing the detected IR radiation for the qualitative and/or quantitative determination of the physiological parameter,

wherein the radiation source (a), the sensing unit (b) and the analyzing unit (c) are incorporated within a casing, and
wherein the casing comprises a first face comprising a screen, wherein the screen is at least partially made of a material, which is optically transparent for NIR/VIS radiation emitted by the radiation source (a) and for IR radiation detected by the sensing unit (b).

According to the present invention, a physiological parameter, namely glucose, is determined by measuring IR radiation emitted from the irradiated body part by an absorption measurement, i.e., the intensity of the emitted IR radiation at the measurement wavelength decreases with an increasing concentration of the physiological parameter and the intensity of the emitted IR radiation at the measurement wavelength increases with a decreasing concentration of the physiological parameter. Thus, the measurement is carried out at least one wavelength or wavelength range where the intensity of the detected IR radiation is inversely dependent from the concentration of the physiological parameter in the bodily fluid of said subject.

According to certain embodiments of the present invention, the IR radiation emitted from the irradiated body part is measured over a time period of at least about 0.5 s, particularly of at least about 1 s to about 120 s and more particularly of at least about 2 s to about 20 s. Thereby, a time-dependent measurement signal is recorded on the basis of which a qualitative and/or quantitative determination of the parameter is performed by the analyzing unit (c).

Further, according to certain embodiments of the present invention, the IR radiation emitted from the irradiated body part is measured over a time period, particularly a time period as described above, wherein the body part is irradiated by VIS/NIR radiation during at least a part of said time period. Particularly, the IR radiation emitted from the irradiated body part is measured over a time period, wherein the temperature of the irradiated body part, more particularly the temperature of the irradiated absorption area in the irradiated body part increases during at least a part of said time period. The increase in temperature may about 2°C to about 10°C, particularly in the range of about 3°C to about 5°C.

Further, according to certain embodiments of the present invention, a temperature-compensated analysis of the detected IR radiation is carried out, wherein the measurement signal is subject to a temperature correction. Particularly, the temperature-compensated analysis of the detected IR radiation is based on a measurement and optionally monitoring of the skin temperature of the irradiated body part. Further, the temperature-compensated analysis may be based on a measurement and optionally monitoring of at least one further temperature such as the environmental temperature, the temperatures of one or more components of the sensing unit, e.g., the temperatures of individual sensors within the sensing unit and/or the temperature of an electronics component of the sensing unit. In certain embodiments, the temperature is monitored over a time period, particularly a time period as described above, during which the IR irradiation emitted from the irradiated body part is measured.

Further, according to certain embodiments of the present invention, the radiation source (a) and the sensing unit (b) are located in positions relative to the irradiated body part, which are defined by an angle of at least 90° or more.

### Detailed description

The present invention involves determination of a physiological parameter, namely glucose, by detecting IR radiation from previously irradiated body parts of a subject, particularly a human subject in the wavelength range of about 5 µm to about 12 µm, particularly in the range of about 8 µm to about 10 µm. More particularly, the present invention involves determination of a physiological parameter by its absorption of IR radiation emitted from a previously irradiated body part of a subject in the wavelength range of about 5 µm to about 12 µm, particularly in the range of about 8 µm to about 10 µm. The signal of the emitted IR radiation at the measurement wavelength decreases with an increasing concentration of the physiological parameter and the signal of the emitted IR radiation at the measurement wavelength increases with a decreasing concentration of the physiological parameter. The physiological parameter may be any compound having characteristic absorption bands in this wavelength range. For example, the physiological parameter is glucose or another clinically relevant analyte such as lactate or troponin.

In a certain embodiment of the invention, the system is adapted for the non-invasive determination of glucose in blood. In this embodiment, IR radiation is detected at a glucose-specific wavelength or wavelength range where glucose has a characteristic absorption band and where the intensity of the detected IR radiation is dependent from the concentration of glucose in the blood. More particularly, the glucose-specific wavelength or wavelength range is selected from a wavelength of about 9.2 µm, a wavelength of about 9.4 µm, a wavelength of about 9.6 µm, a wavelength range comprising at least two of the wavelengths of about 9.2 µm, about 9.4 µm and about 9.6 µm, a wavelength range comprising all three of the wavelengths of about 9.2 µm, about 9.4 µm and about 9.6 µm or any combination thereof. Additionally, IR radiation is detected at a reference wavelength or wavelength range where glucose has no characteristic absorption band and particularly an absorption minimum and where the intensity of the detected IR radiation is substantially independent from the concentration of glucose in blood. More particularly, the reference wavelength or wavelength range is selected from a wavelength or wavelength range between about 8.7 µm to about 9.0 µm, a wavelength or a wavelength range between about 9.7 µm to about 10.2 µm or any combination thereof.

As outlined above, the invention is based on the irradiation of body tissue with electromagnetic radiation in the wavelength range between about 400 nm to about 1500 nm (VIS/NIR radiation) and detection of electromagnetic radiation emitted from the irradiated body part in the wavelength range between about 5 µm to about 15 µm (IR radiation). Irradiation of the body part with VIS/NIR radiation results in an enhanced self-emission of IR radiation from said body part due to local energy absorption, which causes a local increase in temperature. Thus, self-emission of IR radiation from the irradiated body part is increased by previous irradiation of said body part with VIS/NIR radiation. Consequently, irradiation of the body part with an external source of IR radiation in the wavelength range between about 5 µm to about 15 µm is not required. Thus, in certain embodiments, the system of the invention does not include an IR radiation source, particularly in certain embodiments the system of the invention does not include an IR radiation source adapted to irradiate the body part from which the detected IR radiation is emitted.

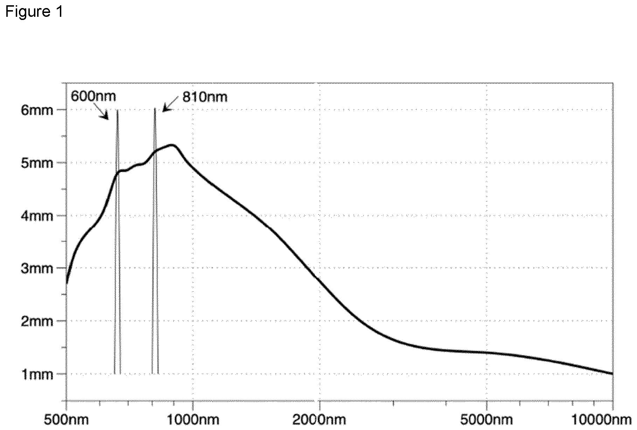

**Figure 1** shows the penetration depth of electromagnetic radiation into body tissue [mm] depending from the wavelength [nm]. It can be seen that the penetration depth is dependent from the wavelength. In the visual (VIS)/near-infrared (NIR) wavelength range between about 400 nm to about 1500 nm, particularly in the range of about 500 nm to about 1500 nm or in the range of about 400 nm to about 1200 nm, more particularly in the range of about 550 nm to about 1200 nm, there is a penetration depth of about 1 mm or more, particularly about 3 mm or more. Thus, a body part irradiated with radiation will absorb electromagnetic energy resulting in a local increase of tissue temperature. This again results in an increased emission of longer-wavelength IR radiation, e.g., IR radiation in the wavelength range of about 5 µm to about 12 µm, where certain organic compounds present in bodily fluids, i.e. physiological parameters, show absorption bands. This allows a quantitative or qualitative determination of such parameters according to the above aspects of the present invention.

In certain embodiments, the VIS/NIR radiation emitted into the body part is in the range of about 550 nm to about 1000 nm, particularly in the range of about 800 nm to about 820 nm, e.g., about 810 nm, and/or in the range of about 590 nm to about 660 nm, e.g., at about 600 nm, and/or in the range of about 920 nm to about 980 nm, e.g., at about 940 nm. In certain embodiments, the VIS/NIR radiation emitted into the body is in the range of about 450 nm to about 800 nm.

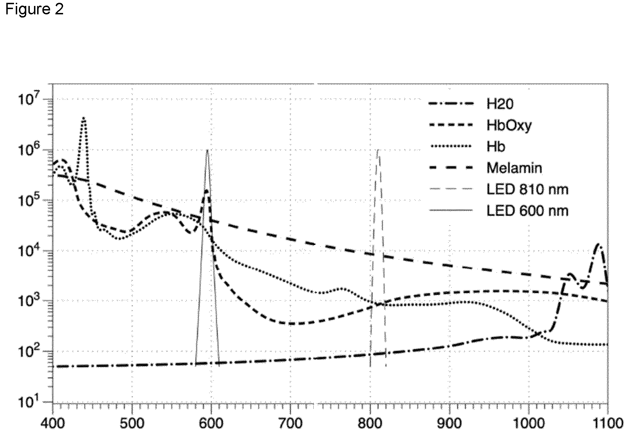

**Figure 2** shows relative absorption coefficients of certain compounds present in the human body depending from the wavelength in the range between 400 nm and 1100 nm. The wavelengths of about 600 nm and about 810 nm, at which radiation may be emitted into the body part, are specifically indicated. In the wavelength range of about 500 nm to about 1050 nm, there is a relatively low absorption of water (H₂O). Further, the major blood constituents hemoglobin (Hb) and oxyhemoglobin (Hboxy) show similar absorption coefficients. The skin pigment melamine shows an absorption coefficient which decreases with increasing wavelength.

The system or device of the invention comprises a radiation source (a) adapted for emitting visual (VIS)/near-infrared (NIR) radiation in the range of about 400 nm to about 1500 nm into a body part of said subject, wherein the body part is particularly selected from a fingertip, an earlobe, a wrist, a forearm, a palm and an upper arm.

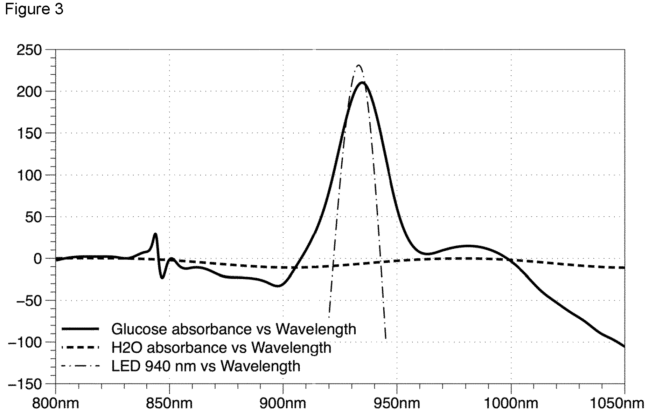

In an embodiment of the invention, the radiation source (a) is adapted for emitting VIS/NIR radiation in the range of about 920 nm to about 960 nm, e.g., about 940 nm into a body part. This irradiation wavelength may be used alone or in combination with at least one further irradiation wavelength. As shown in **Figure 3****,** glucose has an absorption band at a wavelength of 940 nm. Thus, irradiation at a wavelength of about 940 nm leads to a selective excitation of glucose molecules and may result in a stronger absorption of glucose molecules in the IR wavelength range, particularly in the wavelength range of about 5 µm to about 12 µm.

According to an embodiment of the invention, the radiation source (a) is adapted for emitting VIS/NIR radiation in the range of about 920 nm to about 980 nm, e.g., about 940 nm into a body part of said subject, and the sensing unit (b) is further adapted for detecting VIS/NIR radiation having a wavelength of about 940 nm, where the intensity of the detected VIS/NIR radiation is dependent from the concentration of glucose. The measurement signal in the VIS/NIR wavelength range may be combined with the measurement signals in the IR range as described above, e.g., by means of a comparator.

In a still further embodiment, VIS/NIR irradiation occurs at a combination of at least 2 different wavelengths, particularly at a combination of a first wavelength of about 800 nm to about 820 nm, e.g., about 810 nm, and a second wavelength of about 920 nm to about 980 nm, e.g., about 940 nm.

The radiation source (a) is adapted for emitting VIS/NIR radiation in the range of about 400 nm to about 1500 nm, particularly in the range of about 500 nm to about 1500 nm. The VIS/NIR radiation may be emitted continuously or intermittently throughout a predetermined time interval.

The radiation source is adapted to cause a local increase in the temperature of the irradiated body part, e.g., a fingertip, and particularly a local increase in the temperature of the absorption area within the irradiated body part. The local increase in temperature may be in the range of between about 1°C to about 15°C, particularly about 2°C to about 10°C, and more particularly in the range of about 3°C to about 5°C. The locally increased temperature of the irradiated body part, e.g., a fingertip, may be in temperature range up to about 45°C, up to about 40°C or up to about 37°C, for example in the temperature range between about 30°C to about 35°C or about 30°C to about 32°C. This local temperature increase results in an enhanced self-emission of IR radiation from the irradiated body part and particularly from the absorption area within the irradiated body part.

In a certain embodiment, the radiation source (a) may be adapted for emitting radiation continuously at a power of about 10 mW to about 1 W, particularly for about 20 mW to about 500 mW, more particularly of about 50 mW to about 250 mW, and even more particularly of about 100 mW to about 200 mW, e.g. about 150 mW, for a time interval of about 0.1 to about 20 s, particularly of about 0.2 s to about 5 s, and more particularly of about 0.5 s to about 2 s, e.g. about 1 s.

In a further embodiment, the radiation source (a) may be adapted for emitting radiation intermittently at a power of about 10 mW to about 5 W, particularly of about 20 mW to about 1 W, and more particularly of about 50 mW to about 500 mW for a time interval of about 0.1 s to about 20 s, particularly of about 0.2 s to about 5 s, and more particularly of about 0.5 s to about 2 s. The radiation may be emitted intermittently with a pulse frequency of about 1 Hz to about 1 MHz.

In a further embodiment, the radiation source (a) is adapted for emitting radiation continuously or intermittently for a time period of at least about 0.5 s, particularly of at least about 1 s to about 120 s and more particularly of at least about 2 s to about 20 s.

In a further embodiment, the radiation source (a) may be adapted to emit VIS/NIR radiation at a plurality of different wavelengths, e.g., at 2, 3, 4, 5, 6, 7, 8 or even more different wavelengths. For example, the radiation source may be multi-LED chip. The use of a multi-wavelength radiation source allows adjusting a predetermined penetration depth of electromagnetic radiation into the tissue of the irradiated body part depending on specific characteristics of the body part, e.g., pigmentation, skin thickness, presence, or absence of horny skin. As shown in **Figure 1****,** supra, the penetration depth into body tissue varies with the wavelength and the use of VIS/NIR radiation with different wavelengths or with combinations of different wavelengths can be adapted for each subject and/or each body part individually, if desired.

In certain embodiments, the radiation source (a) is a multi-wavelength radiation source is adapted to emit VIS/NIR radiation at several different wavelengths or wavelength ranges, for example, between about 400 nm to about 1200 nm, more particularly between about 450 nm and about 900 nm, e.g., at least 2, 3 4, 6 or 8 wavelengths which may be selected from wavelengths at about 470 nm, about 520 nm, about 590 nm, about 650 nm, about 750 nm and about 810 nm.

The system or device of the invention comprises a sensing unit (b) for detecting emitted IR radiation from the irradiated body part of said subject in the range of about 5 µm to about 12 µm, wherein said sensing unit is adapted for (i) detecting IR radiation having at least one wavelength or wavelength range in the range of about 5 µm to about 12 µm, where the intensity of the detected IR radiation is dependent from the concentration of the physiological parameter in the bodily fluid of said subject, and for (ii) detecting IR radiation having at least one wavelength or wavelength range in the range of about 5 µm to about 12 µm, where the intensity of the detected IR radiation is substantially independent from the concentration of the physiological parameter in the bodily fluid of said subject.

In certain embodiments, the sensing unit (b) is adapted for detecting IR radiation emitted from the irradiated body part over a time period, wherein during at least a part of said time period, e.g. at least about 60% or at least about 80% or at least about 90% of said time period, the body part is irradiated by VIS/NIR radiation, wherein said time period may be at least about 0.5 s, particularly of at least about 1 s to about 120 s and more particularly of at least about 2 s to about 20 s.

In certain embodiments, the sensing unit (b) is adapted for detecting IR radiation emitted from the irradiated body part over a time period, e.g. a time period as described above, wherein during at least a part of said time period, e.g. at least about 60% or at least about 80% or at least about 90% of said time period, the temperature of the irradiated body part, particularly the absorption area, in the irradiated body part is higher than the surrounding tissue, e.g. at least 1°C, at least 2°C, at least 5°C and up to 10°C higher than the surrounding tissue.

In certain embodiments, the sensing unit (b) is adapted for detecting IR radiation emitted from the irradiated body part over a time period, e.g. a time period as described above, wherein during at least a part of said time period, e.g. at least about 60% or at least about 80% or at least about 90% of said time period, the temperature of the irradiated body part, particularly the absorption area, is increasing. The increase in temperature may about 2°C to about 10°C, particularly in the range of about 3°C to about 5°C.

The sensing unit (b) comprises at least one sensor adapted for detecting emitted IR radiation from the irradiated body part. In the sensing unit (b) of the invention, at least one sensor may be an analyte-specific sensor, i.e. a sensor which is adapted for detecting IR radiation having at least one wavelength or wavelength range where the intensity of the detected IR radiation is dependent from the concentration of the physiological parameter in the bodily fluid of said subject, and at least on sensor may be a reference sensor, i.e. a sensor which is adapted for detecting IR radiation having at least one wavelength or wavelength range where the intensity of the detected IR radiation is substantially independent from the concentration of the physiological parameter in the bodily fluid of said subject.

In certain embodiments, the sensing unit (b) is adapted for detecting self-emitted IR radiation from the previously irradiated body part, i.e. IR radiation generated by the body heat of the subject without irradiation by an external IR source. Further, the sensing unit (b) may be adapted for detecting emitted IR radiation from an absorption area within the previously VIS/NIR-irradiated body part wherein the absorption area has a locally increased temperature and exhibits an increased emission of IR radiation in the wavelength range of about 5 µm to about 12 µm.

In certain embodiments, at least one further sensor may be present, e.g. a sensor which (i) is adapted for detecting unspecific IR radiation, (ii) is adapted for detecting unspecific VIS/NIR radiation, (iii) is adapted for detecting VIS/NIR radiation having a wavelength, where the intensity of the detected VIS/NIR radiation is dependent from the concentration of the physiological parameter in the bodily fluid of said subject, and/or (iv) a temperature sensor for measuring the temperature of the body part.

In certain embodiments, the sensing unit (b) is further adapted for a temperature measurement, e.g., with a precision of at least about 1°C, of at least about 0.1°C or of at least about 0.01°C. The sensing unit may be adapted for measuring and optionally monitoring the skin temperature of the irradiated body part and optionally at least one further temperature such as the environmental temperature, the temperatures of individual sensors within the sensing unit (b) and/or the temperature of an electronics component of the sensing unit (b), e.g., the temperature of a circuit board. In those embodiments, the sensing unit (b) may comprise at least one temperature sensor, particularly a plurality of temperature sensors, e.g. 2, 3 or 4 temperature sensors for measuring the skin temperature, and optionally at least one further temperature sensor, e.g., a sensor for measuring the environmental temperature, at least one sensor for measuring the temperatures of individual sensors within the sensing unit and/or a sensor for measuring the temperature of an electronics component of the sensing unit e.g. the temperature of a circuit board of the sensing unit (b).

In the context of the claimed invention, the sensing unit (b) comprises at least one further analyte-specific sensor, i.e., a sensor, which is adapted for detecting VIS/NIR radiation having at least one wavelength or wavelength range where the intensity of the detected VIS/NIR radiation is dependent from the concentration of the physiological parameter in the bodily fluid of said subject. For example, at least one further sensor adapted for detecting VIR/NIR radiation having a wavelength of about 940 nm may be present.

In certain embodiments of the invention, the radiation source (a) and the sensing unit (b) are located in positions relative to the irradiated body part, which are defined by an angle of at least 90° or more. In certain embodiments, the radiation source (a) and the sensing unit (b) are located on opposite sides of the irradiated body part.

The system or device of the invention comprises an analyzing unit (c) for the qualitative and/or quantitative determination of the physiological parameter based on the IR radiation detected in the sensing unit (b). The analyzing unit may comprise, for example, an A/D converter and/or a microcontroller. The analysis of the measured signal may be based on the intensity and/or the decay time.

In particular embodiments, the analyzing unit (c) is adapted for a time-dependent analysis of the detected IR radiation, wherein a measurement signal is recorded over a time period, particularly over a time period of at least about 0.5 s, particularly of at least about 1 s to about 120 s and more particularly of at least about 2 s to about 20 s.

In further particular embodiments, the analyzing unit (c) is adapted for a temperature-compensated analysis of the detected IR radiation. A temperature-compensated analysis comprises a temperature compensation wherein the measurement signal is subject to a temperature correction. Particularly, the temperature compensation is based on the skin temperature of the irradiated body part and optionally at least one further temperature such as the environmental temperature, the temperatures of components of the sensing unit, e.g. the temperatures of individual sensors within the sensing unit and/or the temperature of an electronics component of the sensing unit.

In still further particular embodiments, the analyzing unit (c) is adapted for a time-dependent and temperature-compensated analysis of the detected IR radiation as described above.

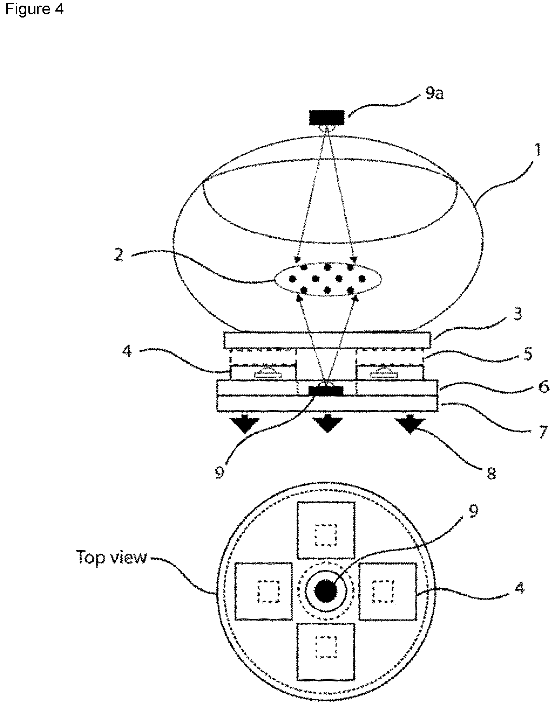

**Figure 4** shows an embodiment of a system of the present invention. A body part (1), e.g., a fingertip, is placed into contact with the system, which is adapted to irradiate an absorption area (2) within the body part (1).

The system comprises a cover (3) which is at least partially made of a material, which is optically transparent. For example, the cover is at least partially made of CaF₂and/or BaF₂or of a plastic material which is transparent in the IR wavelength range of about 5 µm to about 12 µm or a sub-range thereof, e.g., of about 8 µm to about 12 µm, and which is optionally transparent in the VIS/NIR wavelength range of about 400 nm to about 1500 nm or a sub-range thereof. Suitable IR-transparent plastic materials are e.g., the PolyIR plastic materials commercially available from Fresnel Technologies, Fort Worth, Texas, USA. In certain embodiments, the cover may have a thickness of about 0.2 mm to about 2 mm, particularly of about 0.3 mm to about 1.5 mm, more particularly about 1 mm.

The system further comprises at least one sensor (4) which may be provided with a filter element (5) and optionally a lens element (not shown), which e.g., may be arranged between a sensor (4) and a filter element (5). The sensor (4) may be mounted on a circuit board (6). Further, the system comprises at least one radiation source (9, 9a). For example, the system may comprise a radiation source (9) located on the same side as the sensor (4) and/or a radiation source (9a) located on an opposite side of the body part (1) with regard to the sensor (4). If desired, a further sensor (4) may be provided without filter element (5) for monitoring the exact skin temperature of the subject.

The system contains one or more sensors (4). In the embodiment of Figure 4, the system comprises four different sensors (4). The sensor may be an optical detector, particularly an optical photovoltaic detector, e.g., an InAsSb-based detector, which may be used in combination with a lock-in amplifier, if desired. A photovoltaic detector, e.g., an InAsSb-based detector has a rise time of only few nanoseconds and is particularly useful in a set-up wherein the body part is irradiated intermittently. In other embodiments, the sensor may be heat detector, e.g., a thermopile or a bolometer. Suitable sensors include a photovoltaic detector (e.g., Hamamatsu P13894), a thermopile (e.g., Heimann HCS C21 F8-14) or other types of IR sensors (e.g., Sensirion STS21 or Melexis MLX90632). If desired, a sensor (4) may be provided with a filter element (5) capable of selectively transmitting radiation of a desired wavelength or wavelength range. The filter element may have narrow bandwidth, e.g., of about 50 to 100 nm, or a broader bandwidth, e.g., of about 400 nm or more. A filter may be made from germanium or other filter materials, which are optically transmissive for the respective wavelengths. Further, a sensor may be provided with a lens element, e.g., a micro-lens capable of focusing the light falling onto the sensor.

In certain embodiments, the sensor surface may be coated with a noble metal such as Au or Ag, particularly Au, in order to increase its sensitivity. Such a coating which may be shaped as a Bundt baking-pan is described by Awad (Nature Scientific Reports 9:12197 (2019)).

In certain embodiments, the sensor is a miniaturized sensor having an area of about 1 mm² to about 10,000 mm², e.g., of about 10 mm² to about 1,000 mm². In certain embodiments, the sensor may be even more miniaturized, e.g., an ASIC (application-specific integrated circuit).

Further, the device may comprise a circuit board (7) on which the light source (9) is mounted and an active and/or passive heat sink (8).

The VIS/NIR radiation source (9, 9a, 9b) may be adapted for emitting collimated radiation, e.g., a laser-based light source, and/or adapted for emitting non-collimated radiation, e.g., an LED-based light source. For example, the light source may be selected from an LED, a laser diode, a VCSEL (vertical-cavity surface-emitting laser) or a laser. In certain embodiments, a broadband VIS/NIR radiation emitter is used which may be adapted for emitting VIS/NIR radiation in the range of about 650 nm to about 950 nm, particularly in the range of about 750 nm to about 850 nm and more particularly in the range of about 780 nm to about 820 nm. Suitable VIS/NIR emitters are e.g. the OSLON products from Osram such as OSLON SFH 4763.

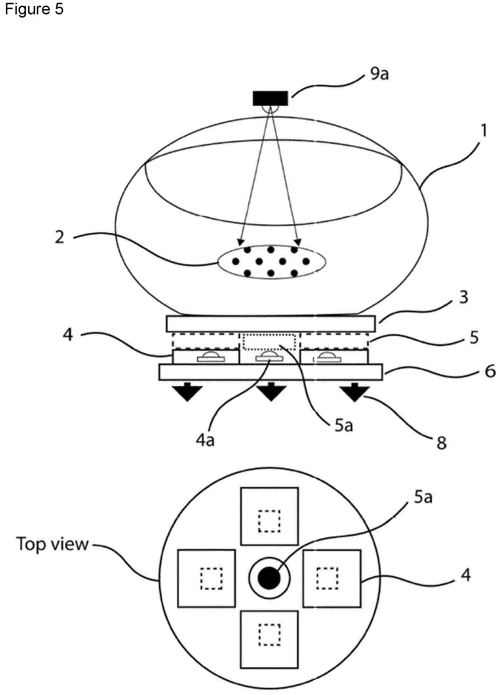

A further embodiment of the system of the invention is shown in **Figure 5****.** Here, a single radiation source (9a) is provided on a side of the body part (1) which is opposite to a sensing unit comprising at least one sensor (4) provided with a filter (5) and a further sensor (4a) provided with a filter (5a). In certain embodiments, sensor (4a) is an optical sensor, e.g., a photodiode. It is adapted for a reference measurement of transmission radiation from radiation source (9a), e.g. for measuring radiation at a wavelength of about 600 nm and/or about 810 nm and/or about 940 nm. For this purpose, filter element (5a) may be a bandpass filter at about 600 nm and/or 810 nm and/or 940 nm.

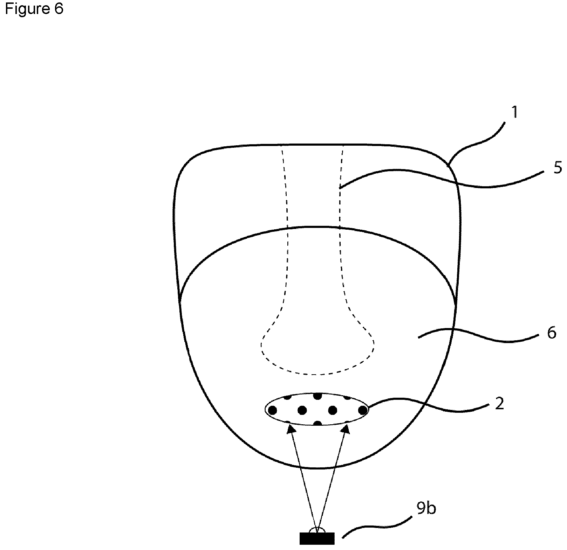

Still a further embodiment of the invention is shown in **Figure 6****.** Here, a radiation source (9b) is provided on a side of the body part (1), e.g., a fingertip, wherein direct access to an absorption area (2) within the body part (1) is provided through the skin of the body part without the radiation passing through a cover structure of the device and/or without passing through a horny structure on the body surface, e.g., a fingernail and/or horny skin. Thereby, interference, e.g., interference from the cover structure or from keratinic horny skin or nail material and optionally nail varnish can be reduced or eliminated. According to this embodiment, a single radiation source (9b) or a plurality of radiation sources (9b), e.g., 2, 3, 4, 6 or 8 radiation sources may be provided at a position around the circumference of the body part (1), e.g., a fingertip. If a plurality of radiation sources is present, they are preferably adapted to emit radiation into a single absorption area (2) within the body part, which may be about 3 mm to about 5 mm below the body surface.

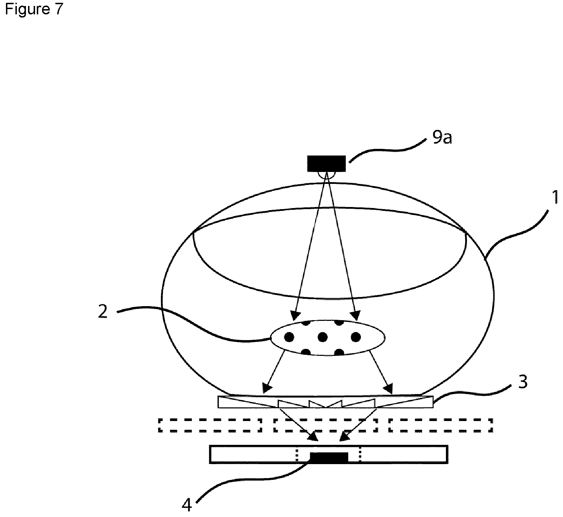

Still a further embodiment of the invention is shown in **Figure 7****.** In this embodiment, a cover (3) is provided which is adapted for focusing IR radiation emitted from the body part to the at least one sensor (4) of the sensing unit. Thereby, the radiation intensity on the sensor and thus the sensitivity and/or accuracy of the measurement may be increased. The cover (3) is made of a material, e.g., plastic, metal, metal oxide or composite material, which is substantially transparent for IR radiation in the wavelength range to be detected on the sensor, particularly for IR radiation in the wavelength range of about 5 µm to about 12 µm or a sub-range thereof, e.g., of about 8 µm to about 12 µm. Suitable materials are e.g., the PolyIR plastic materials, c.f. supra. In this embodiment, the cover (3) may comprise an IR Fresnel lens, i.e., a lens of large aperture and short focal length capable of efficiently focusing IR radiation passing therethrough, or an array comprising a plurality e.g., up to 10 or more IR Fresnel lenses. In certain embodiments, the array may comprise IR Fresnel micro-lenses, e.g., up to 100 or 1000 micro-lenses, which may have diameters in the range of about 50 nm to about 500 µm. In certain embodiments, the IR Fresnel lens may have a back focal length of about 3 mm to about 10 mm, e.g., about 5 mm and may be manufactured from an IR-transparent plastic. For example, a suitable IR Fresnel lens, which is optically transparent in the wavelength range of 8-14 µm is commercially available from Edmund Optics (product family no. 2042).

Further, **Figure 7** shows a radiation source (9a) provided on the opposite side of the body part with regard to the position of the sensing unit, which comprises a sensor (4). It should be noted, however, that one or more radiation sources may also be arranged in a circumferential arrangement around the body part (1), e.g., as shown in Figure 6. It should further be noted, that in this embodiment a plurality of different sensors may be present, e.g., as shown in **Figure 4** and **Figure 5****.**

As shown in **Figure 4** and **Figure 5****,** the system of the invention comprises a plurality of different sensors (4). The system comprises a plurality of analyte-specific, namely glucose-specific sensors, wherein a first sensor is adapted for detecting radiation at a first wavelength or wavelength range, e.g., at a wavelength of about 9.2 µm and at least another first sensor is adapted for detecting IR radiation at a second wavelength range which encompasses the first wavelength or wavelength range and further comprises another wavelength or wavelength range. For example, the other first sensor may be adapted for detecting IR radiation at a wavelength of about 9.2 µm and additionally at a wavelength of about 9.4 µm and/or about 9.6 µm, particularly at a wavelength of about 9.4 µm and a wavelength of about 9.6 µm.

Further, the sensing unit may comprise a plurality of reference sensors adapted for detecting reference radiation at different wavelengths or wavelength ranges. For example, when determining glucose, a reference sensor may be adapted for detecting radiation having a wavelength range between about 8.6 µm and about 9.0 µm. Another reference sensor is adapted for detecting radiation at a wavelength or wavelength range between about 9.8 µm and about 10.2 µm.

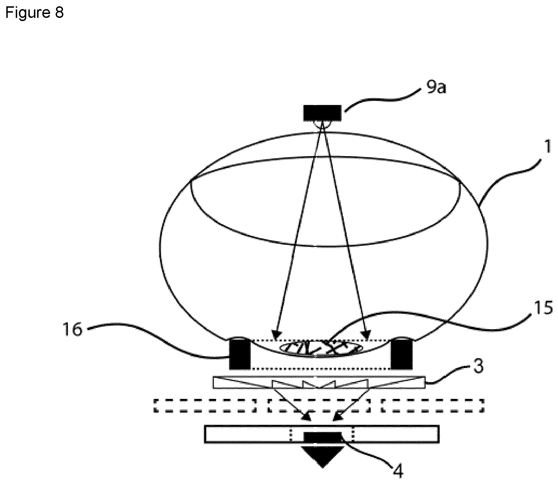

Still a further embodiment of the invention is shown in **Figure 8****.** In this embodiment, a support (16) for the body part (1), e.g., a fingertip, is provided wherein said support (16) comprises an opening adapted to receive a portion (15) of the body part (1). For example, the support may comprise an annular structure with an opening, e.g., a substantially circular opening, in its center. The system is adapted for pressing the body part (1) onto the opening in the support (16) such that a portion (15) of the body part (1), e.g., a portion of the fingertip, is forced into the opening. Thus, the tissue including the blood vessels within portion (15) is compressed resulting in an enhanced amount of capillary blood within portion (15). Thereby, the signal intensity and thus the sensitivity and/or accuracy of the measurement may be increased.

Further, the system of **Figure 8** includes a cover (3) which may be formed as an IR Fresnel lens as described above in the context of **Figure 7****.** It should be noted, however, that other covers are also suitable. Furthermore, a radiation source (9a) is shown which is provided on the opposite side of the body part with regard to the position of the sensing unit, which comprises a sensor (4). It should be noted, however, that one or more radiation sources may also be arranged in a circumferential arrangement around the body part (1), e.g. as shown in **Figure 6****.** It should further be noted, that in this embodiment a plurality of different sensors may be present, e.g., as shown in **Figure 4** and **Figure 5****.**

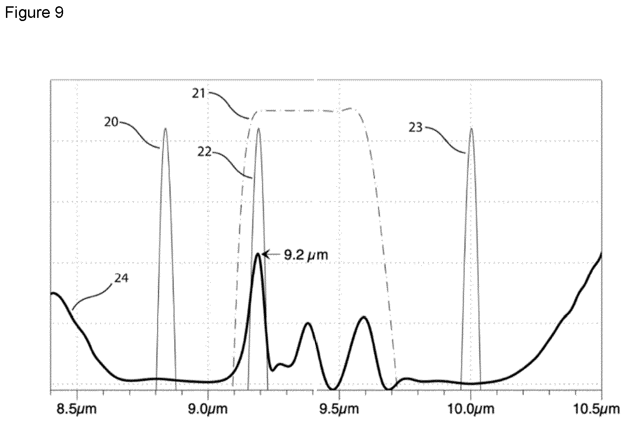

In **Figure 9****,** measurement at a plurality of analyte-specific wavelengths/wavelength ranges and reference wavelengths/wavelength ranges is shown.

The absorption signal of glucose (24) has three different peaks at about 9.2 µm, at about 9.4 µm and about 9.6 µm. A first glucose-specific sensor may be adapted for measuring only the peak at 9.2 µm. Such a sensor would be adapted with a filter element capable of transmitting radiation only in a narrow range (22). Thus, the sensor is capable of selectively detecting radiation within this narrow range. A further glucose-specific sensor may be adapted for measuring radiation at a broader range between about 9.1 µm and about 9.7 µm, thereby encompassing the peaks at about 9.2 µm, 9.4 µm and 9.6 µm. This sensor may be adapted with a filter element capable of transmitting radiation in a broader range (21).

Two reference sensors may be provided, wherein said reference sensors are provided with filter elements capable of transmission of radiation with a wavelength in the range of about 8.6 µm and about 9.0 µm, particularly of about 8.8 µm - 8.9 µm (20) and/or radiation with a wavelength in the range of about 9.8 µm and about 10.2 µm, particularly of about 9.9 µm - 10.1 µm (23), respectively.

Parallel and separate measurements at a wavelength of about 9.2 µm on the one hand and at a wavelength range including the peak at 9.2 µm, but also at least one of the other peaks, particularly the peak at about 9.6 µm have a further advantage, since they allow determination whether the subject's blood contains ethanol. Since ethanol and other alcohols have an absorption band at a wavelength of about 9.6 µm, but not at a wavelength of about 9.2 µm, the ratio between the peak at 9.2 µm and 9.6 µm may be used to determine and optionally correct a disturbance caused by blood alcohol.

In an alternative embodiment, a first glucose-specific sensor may be adapted for measuring only the peak at 9.6 µm. Such a sensor would be provided with a filter element capable of transmitting radiation only in a narrow range. A further glucose-specific sensor may be adapted for measuring radiation at a broader range between about 9.4 µm and about 9.6 µm, thereby encompassing the peaks at about 9.4 µm and about 9.6 µm and not encompassing the peak at 9.2 µm. This sensor may be provided with a filter element capable of transmitting radiation in a broader range.

In a further alternative embodiment, a reference sensor may be provided, which is provided with a filter element capable of transmission of radiation with a wavelength in the range of about 7.8 µm and about 8.2 µm, particularly of about 7.9 µm - 8.1 µm, optionally in combination with at least one further reference sensor, which is provided with a filter element capable of transmission of radiation with a wavelength in the range of about 8.8 µm - 9.2 µm and/or radiation with a wavelength of about 9.8 µm - 10.2 µm, respectively

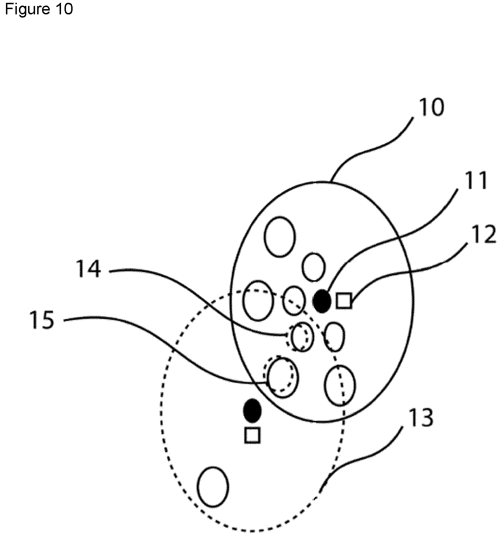

In a still further embodiment of the invention, the system may comprise a sensor, which is adapted for a time-dependent detection of IR radiation having different wavelengths or wavelength ranges. In this embodiment, the system may be provided with a sensor comprising a plurality of filters adapted for transmitting IR radiation having different wavelengths or wavelength ranges wherein said filters may be placed on a sensor during different stages of a measurement cycle thereby allowing detection of different wavelengths or wavelength ranges within a measurement cycle. Such an embodiment is shown in **Figure 10****.** Here, a system is provided comprising a filter wheel (10) capable of rotating around an axis (11) and a shutter wheel (13) capable of rotating around an axis. The filter wheel and the shutter wheel are provided with illumination holes (15) through which light from the radiation source (not shown) may pass into the body part of the subject (not shown). Reflected light from the irradiated body part may pass through different holes (14) of the filter wheel (10) which may be provided with analyte-specific filter elements and/or reference filter elements as described above. The filter wheel's (10) and the shutter wheel's (13) position may be monitored with a magnet (12) in combination with a magnetic sensor. In operation, they may be rotated with predetermined frequencies, thereby allowing time-dependently passing of radiation from the radiation source into the body part and time-dependently passing of radiation emitted from the body part at predetermined time intervals through the different holes (14) of the filter wheel (10) to a sensor (not shown).

In an alternative embodiment (not shown), a sensor which is adapted for a time-dependent detection of IR radiation having different wavelengths or wavelength ranges, may be a Fabry-Perot interferometer, e.g., a MEMS spectrometer for the desired IR wavelength range (cf. Tuohinieni et al., J. Micromech. Microeng. 22 (2012), 115004; Tuohinieni et al., J. Micromech. Microeng. 23 (2013), 075011).

In certain embodiments, the system comprises a single sensor, which is adapted for a time-dependent detection of IR radiation having different wavelengths or wavelength ranges. This sensor may be provided with different filters, e.g. with a filter wheel, or be a Fabry-Perot interferometer as described above.

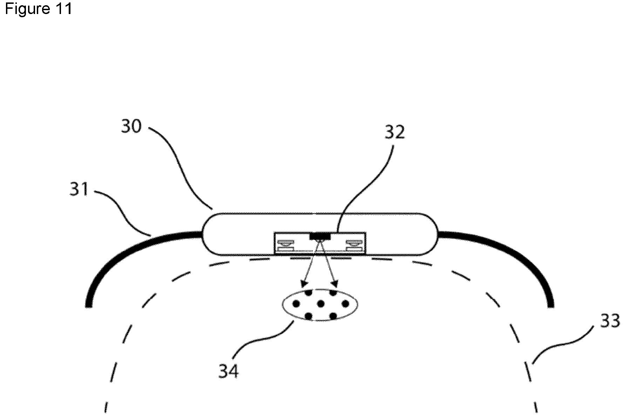

A still further embodiment of the invention is shown in **Figure 11****.** The system of this embodiment is adapted for being permanently fixed to the subject's body. This system is particularly adapted for carrying out a plurality of measurements in predetermined time intervals. The system comprises a housing (30) and a strap (31) for fixing the housing around the body (33), e.g., a wrist or forearm. Further, the system comprises a radiation source for emitting VIS/NIR light into an absorption area (34) of the body part (33) and sensors for detecting IR radiation emitted from the irradiated body part.

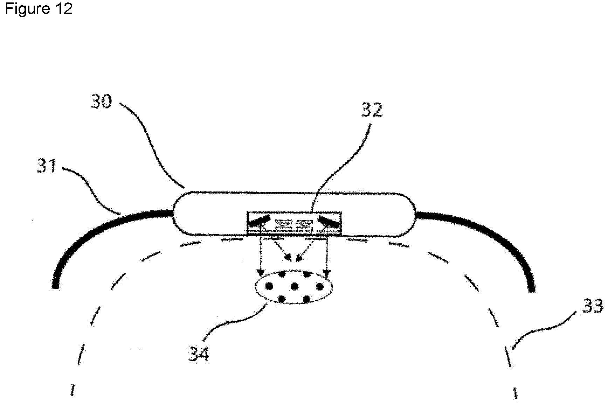

A still further embodiment of the invention is shown in **Figure 12****.** The system of this embodiment is adapted for being permanently fixed to the subject's body and particularly adapted for carrying out a plurality of measurements in predetermined time intervals. The system comprises a housing (30) and a strap (31) for fixing the housing around the body (33), e.g., a wrist or forearm. Further, the system comprises a plurality of radiation sources, e.g., 2 radiation sources, for emitting VIS/NIR light into an absorption area (34) of the body part (33) and sensors for detecting IR radiation emitted from the irradiated body part. The light emitted from these sources may fall at angle, e.g., at an angle of about 30° to about 75° onto the surface of the body part (33).

In **Figure 13****,** a schematic view of the system of **Figure 4** is shown.

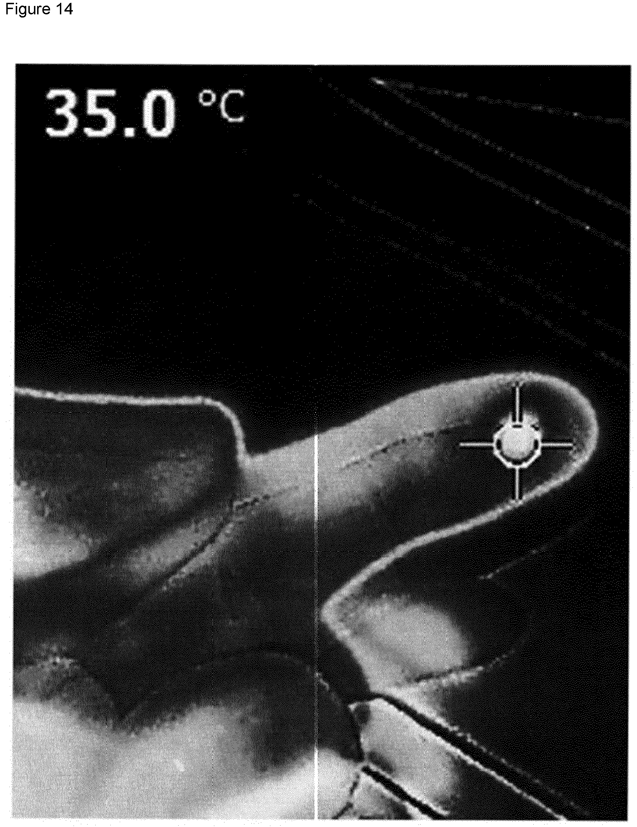

**Figure 14** shows a heat map of a fingertip after irradiation with light of 810 nm for a time period of 2 s.

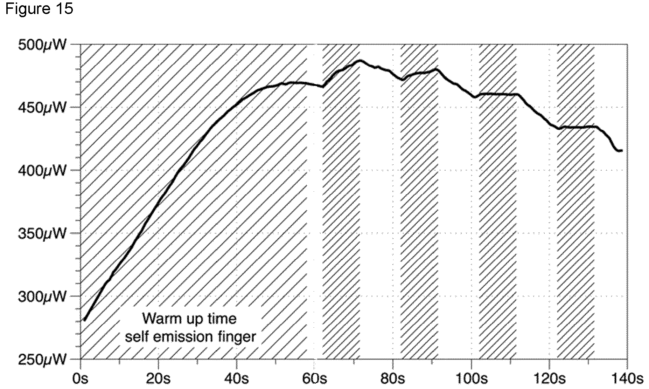

**Figure 15** is diagram showing the time-dependent thermal power output in addition to the self-emission of a fingertip during intermittent irradiation with light of 810 nm with a power of 2 mW and a frequency of 0.1 Hz.

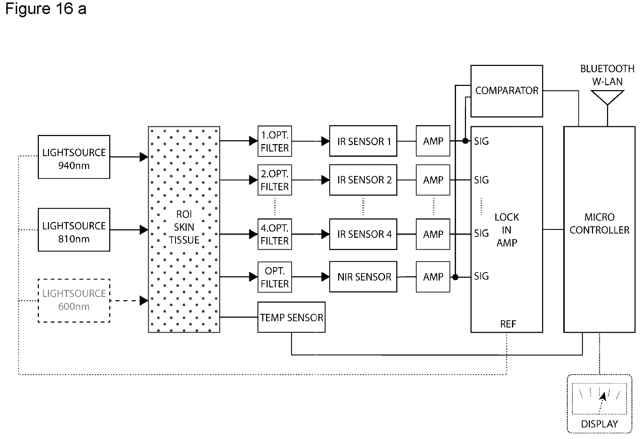

**Figure 16a** shows a block diagram of an embodiment of the sensing unit the present invention. A Region of Interest (ROI), i.e. the skin tissue of a subject, particularly a human subject, is irradiated with a first light source emitting VIS/NIR radiation having a wavelength of 940 nm, a second light source emitting VIS/NIR radiation having a wavelength of about 810 nm and optionally a third light source emitting VIS/NIR radiation having a wavelength of about 600 nm. Radiation transmitted through the Region of Interest or reflected from the Region of Interest is analyzed by a sensing unit. Further, the device comprises a temperature sensor.

The sensing unit comprises a plurality of sensors, for example analyte-specific IR sensors (1) and (2) and reference sensors, e.g., IR sensor (4). For the determination of glucose, an IR sensor (1) may be provided with a first optical filter, which is transmissive for a wavelength of about 9.2 µm and an IR sensor (2) may be provided with a second optical filter, which is transmissive for a wavelength range between about 9.2 µm and about 9.6 µm. A reference sensor (4) may be provided with a fourth optical filter which is transmissive for a wavelength or wavelength range between about 8.6 µm and about 9.0 µm and/or a wavelength or wavelength range between about 9.8 µm and about 10.2 µm. Further, the sensing unit comprises an NIR sensor for detecting VIS/NIR radiation having a wavelength of about 940 µm where glucose has a strong absorption band. The NIR sensor is provided with a suitable optical filter, which is transmissive for this wavelength. Further, the sensing unit may comprise a temperature sensor for measuring the temperature of the skin tissue in the Region of Interest. The respective sensors may be coupled to amplifiers (AMP) for first signal amplification. Signals from individual sensors may be referenced with signals from other sensors by means of a comparator, thereby improving the measurement accuracy and/or signal quality. For example, the measurement signal from the NIR sensor at 940 nm may be referenced with the measurement signal from analyte-specific IR sensor (1). Alternatively or additionally, the measurement signal from the NIR sensor at 940 nm may be referenced with the measurement signals from analyte-specific IR sensor (1) and/or analyte-specific IR sensor (2) and/or reference IR sensor (4). The measured and optionally referenced signals are further amplified by a lock-in amplifier unit and transmitted to a microcontroller unit. A feedback control from the lock-in amplifier to the light sources may be provided. From the microcontroller unit, the signal and/or the result of internal algorithms may be transmitted to a display unit and/or another device, e.g., by a direct connection or via Bluetooth and/or WLAN.

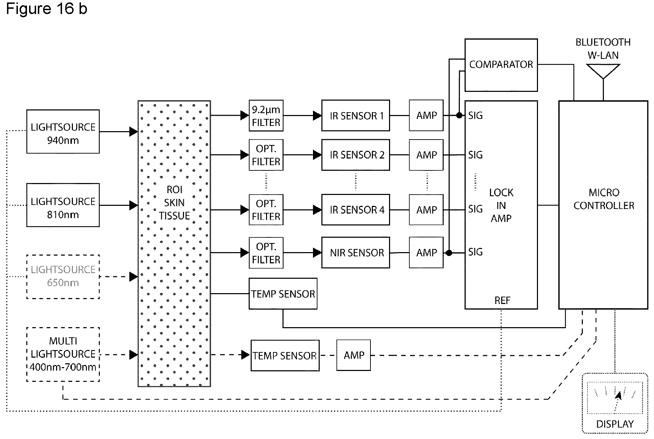

**Figure 16b** shows a block diagram of a further embodiment of the sensing unit of the present invention, which is similar to the sensing unit shown in **Figure** 16a. Here, additionally or alternatively, a multi-wavelength light source, e.g., a multi-wavelength LED comprising a plurality of individual diodes is provided. The multi-wavelength light source may e.g., have a wavelength range from 400 nm to about 700 nm is provided and may be operated by the microcontroller unit. Further, a temperature sensor coupled to an amplifier (AMP) is present. This temperature sensor may also be operated by the microcontroller unit.

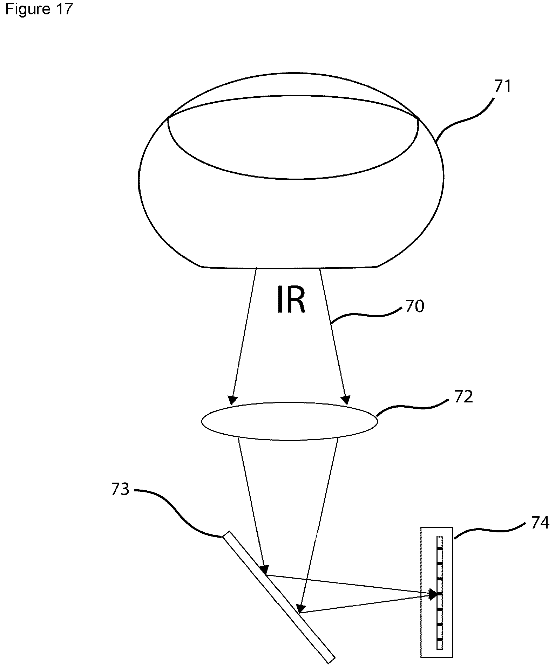

In a still further embodiment of the invention, the system may comprise a spectral or line sensor or spectral or line sensor array, typically a bolometer or thermopile array, which is adapted for detecting an IR spectrum within the wavelength range of interest, e.g., including the range of about 7 µm to about 12 µm, particularly including the range of about 8 µm to about 10 µm. An IR spectrum may be generated by passing the IR radiation from the irradiated body part through a spectral splitting or diffracting device and then to the sensor or sensor array. Such an embodiment is shown in **Figure 17****.** Emitted IR radiation (70) from the irradiated body part (71), e.g., a fingertip, is optionally focused by a focusing element (72) adapted for focusing IR radiation, e.g., a lens or concave mirror element, and then passed to a spectral splitting or diffracting element (73), e.g., a prism or a transmissive or reflective optical grating, where the IR radiation is split according to its wavelength. From there the diffracted radiation is passed to a spectral sensor or line sensor or sensor array (74), typically a bolometer or thermopile array, where an IR spectrum in the wavelength range of interest, e.g., between 8 µm and about 20 µm including analyte-specific wavelengths or wavelength ranges and reference wavelengths or wavelength ranges, e.g., as described above, is detected. The amount of the physiological parameter of interest, e.g., glucose may be determined by spectral analysis according to the relative intensities of predetermined analyte-specific and reference wavelengths.

The system and the method of the invention allow qualitative and/or quantitative determination of the physiological parameter to be measured, particularly qualitative and/or quantitative determination of glucose in blood.

In certain embodiments, the concentration of the physiological parameter, e.g., the concentration of glucose in blood, is quantitatively determined. In certain embodiments, the alteration rate of the measured amount of the physiological parameter, e.g., glucose, is determined. These embodiments may involve a non-quantitative measurement, e.g., a relative measurement of the alteration of the analyte amount per time unit, i.e., the increase of the analyte amount or the decrease of the analyte amount per time unit. In case the alteration of the analyte amount into a single direction, i.e., increase or decrease, exceeds a predetermined level and/or time period, the system will provide an alert. This embodiment is particularly useful for systems as shown in **Figure 11** and **Figure 12,** which may be permanently fixed at the body of the subject, e.g., around the wrist, forearm, or upper arm. This embodiment may be adapted for steady glucose level monitoring.

In certain embodiments, the system of the invention is adapted for performing both non-quantitative measurements and quantitative measurements. For example, the system may be adapted for performing non-quantitative measurements, e.g., qualitatively measuring the alteration, e.g., the increase or decrease, of the analyte amount over time during standard operation. Non-quantitative measurements may e.g., be performed as continuous and/or intermittent monitoring measurements, as required. In case the alteration of the analyte amount exceeds a predetermined level and/or time period, the system is adapted to switch to a quantitative measurement in order to provide more detailed information. In these embodiments, a system adapted to be permanently fixed to the body, e.g., to an arm wrist, or to an ankle, may be used. Specific embodiments of such systems are shown in **Figure 11** and **Figure 12****.**

In certain embodiments, the system is adapted to perform non-quantitative measurements, e.g., continuous and/or intermittent monitoring measurements, and quantitative measurements on several different body parts. For example, the system may be adapted to perform non-quantitative measurements on a first body part, e.g., a body part to which the system may be permanently fixed, such as an arm wrist or an ankle, and to perform quantitative measurements on a second body part, e.g., a body part where capillary vessels are more accessible, such as an earlobe, a palm or a fingertip. For performing a measurement on the second body part, the system is removed from the first body part and brought into contact, particularly into direct contact with the second body part. After performing the measurement on the second body part, the system may be removed therefrom and brought again into contact with the first body part, e.g., by fixing the system to the first body part. In specific embodiments, the first body part is an arm wrist and/or the second body part is a fingertip.

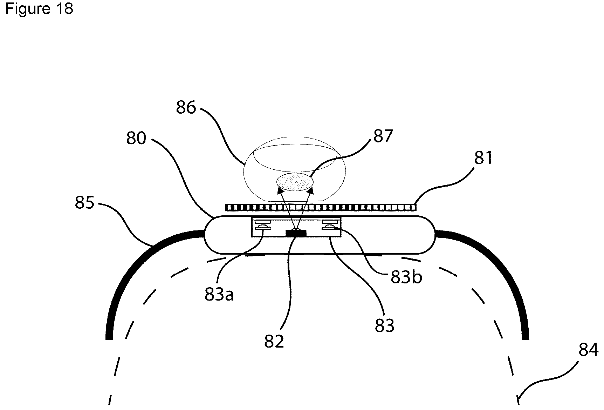

A still further embodiment of the invention is shown in **Figure 18****.** Here, a device comprising a non-invasive system for determining a physiological parameter, e.g., glucose, in a bodily fluid of a subject is shown. The device comprises a casing (80), which comprises a first face comprising a screen (81) which is at least partially made of a material which is optically transparent for NIR/VIS radiation emitted by a radiation source (82). Further, the device comprises a sensing unit (83) comprising at least two sensors (83a), (83b).

The device (80) may be a mobile device, e.g., a smart phone, a smart watch, a tablet or a fitness-tracker device. The device may be worn on the body of a subject, e.g., a wrist (84), and may be fixed by a band (85), e.g., a wrist band. A body part (86), e.g., a fingertip (or, alternatively, a plurality of fingertips or a palm) is placed upon screen (81) for measurement. An absorption area (87) within body part (86) is irradiated by VIS/NIR radiation emitted by radiation source (82). The irradiated body part absorbs electromagnetic energy resulting in a local increase of local temperature and in an increased emission of IR radiation from the absorption area in the wavelength range of about 5 µm to about 12 µm. Molecules of the detected physiological parameter, e.g., glucose molecules, absorb the emitted IR radiation, present in the tissue of body part (86). Thus, at wavelengths or wavelength ranges corresponding to the absorption bands of the physiological parameter of interest, the signal of the emitted IR radiation decreases with an increasing concentration of the physiological parameter and the signal of the emitted IR radiation increases with a decreasing concentration of the physiological parameter.

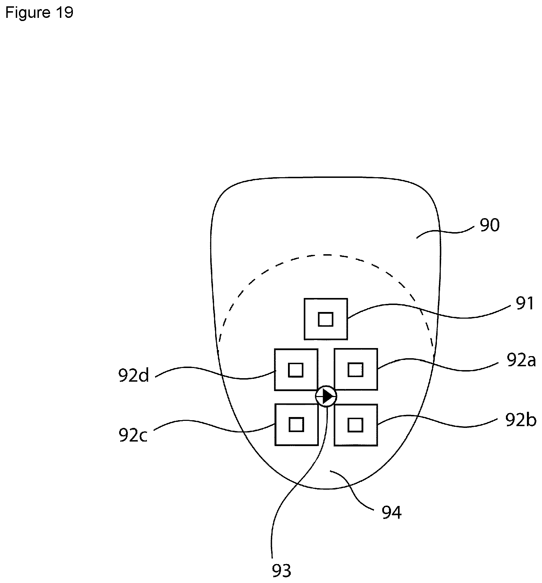

**Figure 19** shows a device of the invention comprising a VIS/NIR radiation source (93), e.g., an LED, adapted for irradiating a body part, e.g., the fingertip (94) of a finger (90). The device comprises an IR temperature sensor (91), e.g., a bolometer, for measuring the temperature of the body part. Further, the device comprises four IR sensors (92a, 92b, 92c, 92d) provided with optical filters adapted for wavelength-specific measurement of the analyte to be determined, e.g., glucose. IR sensors (92a, 92b) may be adapted for measurements at analyte-specific wavelengths or wavelength ranges and IR sensors (92c, 92d) may be adapted for measurements at reference wavelengths or wavelength ranges. For example, sensor (92a) may be provided with an optical filter which is transmissive for a glucose-specific wavelength of about 9.2 µm and sensor (92b) may be provided with an optical filter, which is transmissive for a glucose-specific wavelength range between about 9.2 µm and about 9.6 µm, sensor (92c) may be provided with an optical filter which is transmissive for a reference wavelength or wavelength range between about 8.6 µm and about 9.0 µm and sensor (92d) may be provided with an optical filter which is transmissive for a reference wavelength or a wavelength range between about 9.8 µm and about 10.2 µm.

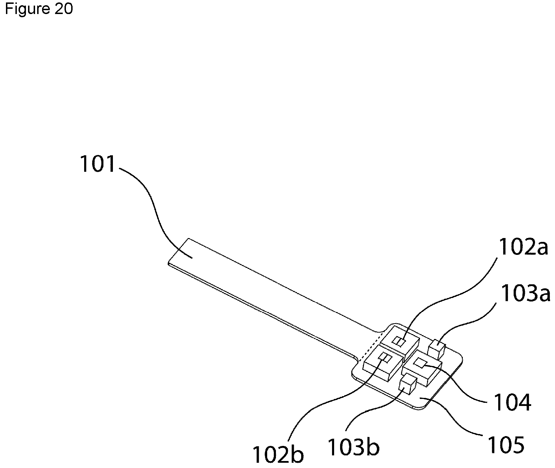

**Figure 20** shows a further device of the present invention comprising a board (105) on which at least one VIS/NIR radiation source, e.g., two radiation sources (103a, 103b) and sensors (102a, 102b, 104) are mounted. The board is connected via means (101), e.g., a printed circuit (PCB) or printed circuit board assembly (PCBA), particularly a flexible or starrflex PCB or PCBA, to an analog-digital converter, a microcontroller or a processor (not shown). Radiation sources (103a, 103b) may be adapted to emit VIS/NIR radiation at the same wavelengths or at different wavelengths. For example, both radiation sources may emit radiation of a wavelength of about 810 µm or radiation at a wavelength of about 940 µm. Alternatively, one of the radiation sources may be adapted to emit radiation at a wavelength of about 810 µm and the other radiation source may be adapted to emit irradiation at a wavelength of about 940 µm. The device comprises an IR temperature sensor (104), e.g., a bolometer, for measuring the temperature of a body part (not shown). Further, the device comprises two IR sensors (102a, 102b) each comprising two separate sensing chips. Each sensing chip may be provided with a different optical filter. IR sensor (102a) may be adapted for measurements at analyte-specific wavelengths or wavelength ranges and IR sensor (102b) may be adapted for measurements at reference wavelengths or wavelength ranges. For example, a first chip on sensor (102a) may be provided with an optical filter which is transmissive for a glucose-specific wavelength of about 9.2 µm and a second chip on sensor (102a) may be provided with an optical filter, which is transmissive for a glucose-specific wavelength range between about 9.2 µm and about 9.6 µm, a first chip on sensor (102b) may be provided with an optical filter which is transmissive for a reference wavelength or wavelength range between about 8.6 µm and about 9.0 µm and a second chip on sensor (102b) may be provided with an optical filter which is transmissive for a reference wavelength or a wavelength range between about 9.8 µm and about 10.2 µm. In a further miniaturized embodiment, the device may comprise a single sensor step comprising at least 4 chips with 4 different optical filters (not shown). In a still further miniaturized embodiment, the device may be a single unit comprising all chips, all filters, all radiation sources and even the microelectronic components as analog-digital converters and microcontrollers in a single application-specific integrated circuit (ASIC) (not shown).

The device shown in **Figure 20** (or any other device of the invention) may be provided as component for integration into a multi-function device, e.g., a smart device, such as a smart watch or a mobile phone. Alternatively, the device may be provided as stand-alone device.

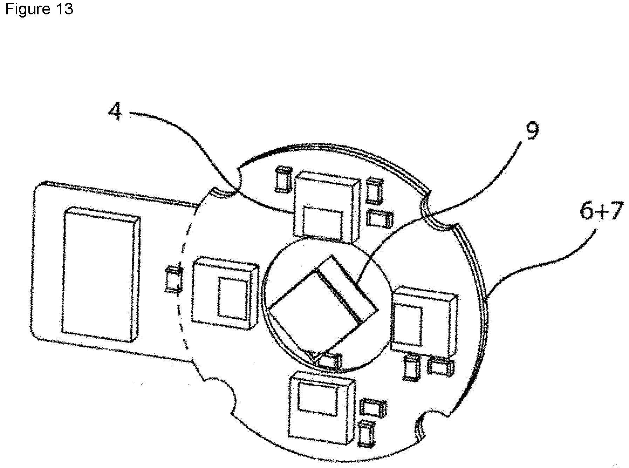

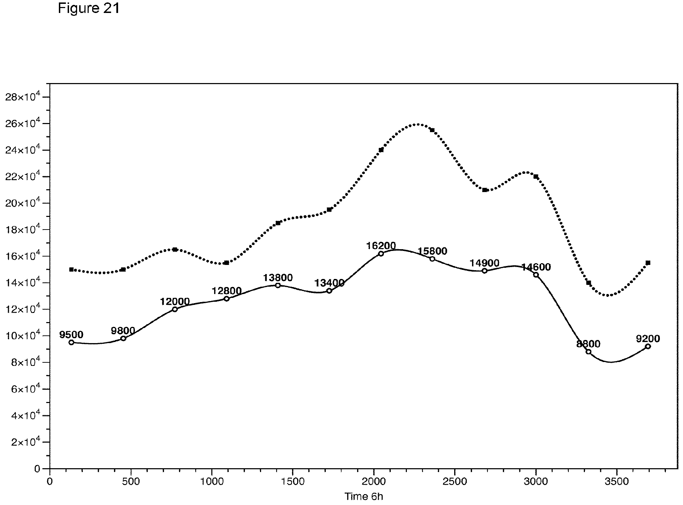

**Figure 21** shows a comparison of the glucose concentration measured by an invasive method (straight line), i.e., an amperometric measurement of blood samples with a conventional glucometer, and the relative (non-calibrated) glucose amount measured by the non-invasive method of the invention (dotted line), i.e., a measurement with a device as shown in **Figure 13****.** The measurement was performed over a time period of 6 h. A high correlation between the conventional invasive glucose measurement and the inventive non-invasive glucose measurement was observed.

A still further aspect of the invention is a non-invasive system for determining glucose in blood, which allows identification and optional correction of disturbances caused by blood alcohol comprising a sensing unit for detecting emitted IR radiation from a body part of said subject in the range of about 5 µm to about 12 µm,
wherein said sensing unit is adapted for (i) detecting IR radiation at a wavelength of about 9.2 µm and separately therefrom for detecting IR radiation at a wavelength of at least about 9.2 µm and about 9.6 µm, particularly at a wavelength range encompassing the wavelength of about 9.2 µm, about 9.4 µm and about 9.6 µm, and an analyzing unit for the separate determination of glucose from the above sensing units.

Still a further aspect of the invention is a method for non-invasively determining glucose in blood of a subject using this system.

Preferred features of these aspects are as previously indicated in the above specification.

Still a further aspect of the present invention is the use of an InAsSb sensor, optionally in combination with a lock-in amplifier for the measure of IR radiation emitted from a body part.

Preferred features of this aspect are as previously indicated in the above specification.

Still a further aspect of the present invention is a system and method for the non-quantitative measurement of glucose involving a plurality of measurements during a predetermined time interval and determining an alteration of the measurement signal indicating an alteration of the amount of analyte and providing an alter if the alteration of the glucose amount to one direction, i.e., increase or decrease, exceeds a certain level in a predetermined time period. This system and method may be adapted for steady glucose level monitoring.

Preferred features of this aspect are as previously indicated in the above specification. The invention is defined by the appended claims.

## Claims

1. A device comprising a non-invasive system for determining glucose in a bodily fluid of a subject, wherein the device comprises a casing (80), wherein the device includes:
(a) a radiation source (82) adapted for emitting visual (VIS)/near-infrared (NIR) radiation in the range of about 400 nm to about 1500 nm into a body part (86) of said subject, wherein the body part (86) is particularly selected from a fingertip, a plurality of finger tips, and a palm, and wherein the radiation source (82) is further adapted that the irradiated body part (86) absorbs electromagnetic energy resulting in a local increase of tissue temperature and in an increased emission of **IR** radiation in the wavelength range of about 5 µm to about 12 µm,
(b) a sensing unit (83) for detecting emitted IR radiation from the previously irradiated body part of said subject in the range of about 5 µm to about 12 µm,
wherein said sensing unit (83) is adapted for (i) detecting IR radiation having at least one wavelength or wavelength range where the intensity of the detected IR radiation is dependent from the concentration of glucose in the bodily fluid of said subject,
wherein the intensity of the emitted IR decreases with an increasing concentration of glucose and the intensity of the emitted IR radiation increases with a decreasing concentration of glucose,
wherein the sensing unit (83) comprises a plurality of glucose-specific sensors wherein a first sensor is adapted for detecting IR radiation at a first wavelength of about 9.2 µm and at least one other first sensor is adapted for detecting IR radiation at a second wavelength range which encompasses the first wavelength and further encompasses another wavelength or wavelength range, and
wherein the sensing unit (83) is adapted for (ii) detecting IR radiation having at least one wavelength or wavelength range where the intensity of the detected IR radiation is substantially independent from the concentration of glucose in the bodily fluid of said subject, and
(c) an analyzing unit for the qualitative and/or quantitative determination of glucose based on the IR radiation detected in the sensing unit (b), and
wherein the casing (80) comprises a first face comprising a screen (81), wherein the screen (81) is at least partially made of a material, which is optically transparent for NIR/VIS radiation emitted by the radiation source (a) (82) and for IR radiation detected by the sensing unit (b) (83), wherein the radiation source (a) (82), the sensing unit (b) (83) and the analyzing unit (c) are incorporated within the casing (80).

2. The device of claim 1, wherein the at least one other first glucose-specific sensor of the sensing unit (83) is adapted for detecting IR radiation at a wavelength of about 9.2 µm and additionally at a wavelength of about 9.4 µm and/or about 9.6 µm, particularly at a wavelength of about 9.4 µm and about 9.6 µm.

3. The device of claim 1 or 2,
wherein the radiation source (a) (82) is adapted for emitting radiation through the screen (81), and/or
wherein the sensing unit (b) (83) is adapted for detecting radiation entering the casing (80) through the screen (81), and/or
wherein the device is a mobile device, and which is particularly selected from a smart phone, a smart watch, a tablet and a fitness tracker device, and/or
wherein the optically transparent material is selected from an inorganic material such as CaF₂ and/or BaF₂ and from an organic material such as a plastic material, and/or
wherein the optically transparent material has a thickness of about 0.2 mm to about 2 mm, particularly of about 0.3 mm to about 1 mm.

4. The device of any one of one of claims 1-3, wherein the bodily fluid is blood.

5. The device of any one of one of claims 1-4,
which further comprises a lens element adapted for focusing IR radiation from the body part to the sensing unit (b), particularly to the at least one sensor of the sensing unit (b),
wherein the lens element is incorporated within the casing (80) and particularly wherein the lens element comprises an IR Fresnel lens or an array comprising a plurality of IR Fresnel lenses.

6. The device of any one of one of claims 1-5, wherein the sensing unit (b) (83) is adapted for detecting IR radiation emitted from the irradiated body part over a time period, wherein the body part is irradiated by VIS/NIR radiation during at least a part of said time period, and/or wherein the analyzing unit (c) is adapted for a time-dependent analysis of the detected IR radiation, wherein a measurement signal is recorded over a time period.

7. Use of the device of any one of claims 1-6 for non-invasively determining glucose in a bodily fluid of a subject, wherein the bodily fluid is blood, and wherein the alteration rate of the amount of glucose in blood is determined.

8. A method for non-invasively determining glucose in a bodily fluid of a subject comprising the steps:
(a) irradiating a body part of said subject with visual (VIS)/near-infrared (NIR) radiation in the wavelength range of about 400 nm to about 1500 nm, or about 500 nm to about 1500 nm, wherein the irradiated body part absorbs electromagnetic energy resulting in a local increase of tissue temperature and in an increased emission of IR radiation in the wavelength range of about 5 µm to about 12 µm,
(b) detecting emitted IR radiation from the previously irradiated body part of said subject in the wavelength range of about 5 µm to about 12 µm,
comprising separately (i) detecting IR radiation having at least two wavelengths or wavelength ranges where the intensity of the detected IR radiation is dependent from the concentration of glucose in the bodily fluid of said subject,
wherein the intensity of the emitted IR radiation decreases with an increasing concentration of glucose and the intensity of the emitted IR radiation increases with a decreasing concentration of glucose, and
(ii) detecting IR radiation having at least one wavelength or wavelength range where the intensity of the detected IR radiation is substantially independent from the concentration of glucose in the bodily fluid of said subject,
comprising detecting IR radiation with a plurality of glucose-specific sensors wherein a first sensor is adapted for detecting IR radiation at a first wavelength of about 9.2 µm and at least one other first sensor is adapted for detecting IR radiation at a second wavelength range which encompasses the first wavelength and further encompasses another wavelength or wavelength range, and
(c) analyzing the detected IR radiation for the qualitative and/or quantitative determination of glucose,
wherein the radiation source (a), the sensing unit (b) and the analyzing unit (c) are incorporated within a casing , and
wherein the casing comprises a first face comprising a screen, wherein the screen is at least partially made of a material, which is optically transparent for NIR/VIS radiation emitted by the radiation source (a) and for IR radiation detected by the sensing unit (b),
wherein optionally step (b) further comprises (iii) carrying out a temperature measurement, and step (c) optionally further comprises carrying a temperature-compensated analysis of the detected IR radiation, and
wherein the concentration of glucose is quantitatively determined, and/or wherein the alteration rate of the amount of glucose is determined, particularly non-quantitatively determined.

9. The method of claim 8,
wherein the at least one other first glucose-specific sensor of the sensing unit is adapted for detecting IR radiation at a wavelength of about 9.2 µm and additionally at a wavelength of about 9.4 µm and/or about 9.6 µm, particularly at a wavelength of about 9.4 µm and about 9.6 µm.

10. The method of claim 8 or 9,
wherein the bodily fluid is blood.

## Patentansprüche

1. Vorrichtung umfassend ein nicht-invasives System zur Bestimmung von Glukose in einer Körperflüssigkeit eines Individuums, wobei die Vorrichtung ein Gehäuse (80) umfasst, wobei die Vorrichtung umfasst:
(a) eine Strahlungsquelle (82), welche dazu eingerichtet ist, sichtbare (VIS)/nahinfrarote (NIR) Strahlung im Bereich von etwa 400 nm bis etwa 1500 nm in einen Körperteil (86) des Individuums zu emittieren, wobei der Körperteil (86) insbesondere ausgewählt ist aus einer Fingerspitze, einer Mehrzahl von Fingerspitzen und einer Handfläche, und wobei die Strahlungsquelle (82) ferner dazu eingerichtet ist, dass der bestrahlte Körperteil (86) elektromagnetische Energie absorbiert, was zu einer lokalen Erhöhung der Gewebetemperatur und zu einer erhöhten Emission von IR-Strahlung im Wellenlängenbereich von etwa 5 µm bis etwa 12 µm führt,
(b) eine Erfassungseinheit (83) zum Detektieren emittierter IR-Strahlung aus dem zuvor bestrahlten Körperteil des Individuums im Bereich von etwa 5 µm bis etwa 12 µm,
wobei die Erfassungseinheit (83) dazu eingerichtet ist, (i) IR-Strahlung mit mindestens einer Wellenlänge oder einem Wellenlängenbereich zu detektieren, bei welchem die Intensität der detektierten IR-Strahlung von der Glukosekonzentration in der Körperflüssigkeit des Individuums abhängt,
wobei die Intensität des emittierten IR mit einer zunehmenden Glukosekonzentration abnimmt und die Intensität der emittierten IR-Strahlung mit einer abnehmenden Glukosekonzentration zunimmt,
wobei die Erfassungseinheit (83) eine Mehrzahl von glukosespezifischen Sensoren umfasst, wobei ein erster Sensor dazu eingerichtet ist, IR-Strahlung bei einer ersten Wellenlänge von etwa 9,2 µm zu detektieren, und mindestens ein anderer erster Sensor dazu eingerichtet ist, IR-Strahlung bei einem zweiten Wellenlängenbereich zu detektieren, welcher die erste Wellenlänge umfasst und ferner eine andere Wellenlänge oder einen anderen Wellenlängenbereich umfasst, und
wobei die Erfassungseinheit (83) dazu eingerichtet ist, (ii) IR-Strahlung mit mindestens einer Wellenlänge oder einem Wellenlängenbereich zu detektieren, bei welchem die Intensität der detektierten IR-Strahlung im Wesentlichen unabhängig von der Glukosekonzentration in der Körperflüssigkeit des Individuums ist, und
(c) eine Analyseeinheit für die qualitative und/oder quantitative Bestimmung von Glukose auf der Grundlage der in der Erfassungseinheit (b) detektierten IR-Strahlung, und
wobei das Gehäuse (80) eine erste Fläche umfassend einen Bildschirm (81) umfasst, wobei der Bildschirm (81) mindestens teilweise aus einem Material hergestellt ist, welches für von der Strahlungsquelle (a) (82) emittierten NIR/VIS-Strahlung und für von der Erfassungseinheit (b) (83) detektierte IR-Strahlung optisch transparent ist, wobei die Strahlungsquelle (a) (82), die Erfassungseinheit (b) (83) und die Analyseeinheit (c) innerhalb des Gehäuses (80) integriert sind.

2. Vorrichtung nach Anspruch 1, wobei der mindestens eine andere erste glukosespezifische Sensor der Erfassungseinheit (83) dazu eingerichtet ist, IR-Strahlung bei einer Wellenlänge von etwa 9,2 µm und zusätzlich bei einer Wellenlänge von etwa 9,4 µm und/oder etwa 9,6 µm zu detektieren, vorzugsweise bei einer Wellenlänge von etwa 9,4 µm und etwa 9,6 µm.

3. Vorrichtung nach Anspruch 1 oder 2,
wobei die Strahlungsquelle (a) (82) dazu eingerichtet ist, Strahlung durch den Bildschirm (81) zu emittieren, und/oder
wobei die Erfassungseinheit (b) (83) dazu eingerichtet ist, Strahlung zu detektieren, welche durch den Bildschirm (81) in das Gehäuse (80) eintritt, und/oder
wobei die Vorrichtung eine Mobilvorrichtung ist, und welche insbesondere ausgewählt ist aus einem Smartphone, einer Smartwatch, einem Tablet und einer Fitness-Tracker-Vorrichtung, und/oder
wobei das optisch transparente Material ausgewählt ist aus einem anorganischen Material wie CaF₂ und/oder BaF₂ und aus einem organischen Material wie einem Kunststoff, und/oder
wobei das optisch transparente Material eine Dicke von etwa 0,2 mm bis etwa 2 mm aufweist, insbesondere von etwa 0,3 mm bis etwa 1 mm.

4. Vorrichtung nach einem der Ansprüche 1-3, wobei die Körperflüssigkeit Blut ist.

5. Vorrichtung nach einem der Ansprüche 1-4,
welche ferner ein Linsenelement umfasst, welches dazu eingerichtet ist, IR-Strahlung von dem Körperteil auf die Erfassungseinheit (b) zu fokussieren, insbesondere auf den mindestens einen Sensor der Erfassungseinheit (b),
wobei das Linsenelement innerhalb des Gehäuses (80) integriert ist und insbesondere wobei das Linsenelement eine IR-Fresnellinse oder eine Anordnung umfasst, welche eine Mehrzahl von IR-Fresnellinsen umfasst.

6. Vorrichtung nach einem der Ansprüche 1-5, wobei die Erfassungseinheit (b) (83) dazu eingerichtet ist, IR-Strahlung zu detektieren, welche von dem bestrahlten Körperteil über einen Zeitraum emittiert wird, wobei der Körperteil während mindestens eines Teils des Zeitraums mit VIS/NIR-Strahlung bestrahlt wird, und/oder
wobei die Analyseeinheit (c) für eine zeitabhängige Analyse der detektierten IR-Strahlung eingerichtet ist, wobei ein Messsignal über einen Zeitraum aufgezeichnet wird.

7. Verwendung der Vorrichtung nach einem der Ansprüche 1-6 zur nicht-invasiven Bestimmung von Glukose in einer Körperflüssigkeit eines Individuums, wobei die Körperflüssigkeit Blut ist und wobei die Änderungsrate der Glukosemenge im Blut bestimmt wird.

8. Verfahren zur nicht-invasiven Bestimmung von Glukose in einer Körperflüssigkeit eines Individuums, umfassend die Schritte:
(a) Bestrahlen eines Körperteils des Individuums mit sichtbarer (VIS)/nahinfraroter (NIR) Strahlung im Wellenlängenbereich von etwa 400 nm bis etwa 1500 nm oder etwa 500 nm bis etwa 1500 nm, wobei der bestrahlte Körperteil elektromagnetische Energie absorbiert, was zu einer lokalen Erhöhung der Gewebetemperatur und zu einer erhöhten Emission von IR-Strahlung im Wellenlängenbereich von etwa 5 µm bis etwa 12 µm führt,
(b) Detektieren von emittierter IR-Strahlung aus dem zuvor bestrahlten Körperteil des Individuums im Wellenlängenbereich von etwa 5 µm bis etwa 12 µm,
umfassend separates (i) Detektieren von IR-Strahlung mit mindestens zwei Wellenlängen oder Wellenlängenbereichen,
bei welchen die Intensität der detektierten IR-Strahlung von der Glukosekonzentration in der Körperflüssigkeit des Individuums abhängt,
wobei die Intensität der emittierten IR-Strahlung mit einer zunehmenden Glukosekonzentration abnimmt und die Intensität der emittierten IR-Strahlung mit einer abnehmenden Glukosekonzentration zunimmt, und
(ii) Detektieren von IR-Strahlung mit mindestens einer Wellenlänge oder einem Wellenlängenbereich, bei welchem die Intensität der detektierten IR-Strahlung im Wesentlichen unabhängig von der Glukosekonzentration in der Körperflüssigkeit des Individuums ist, umfassend Detektieren von IR-Strahlung mit einer Mehrzahl von glukosespezifischen Sensoren, wobei ein erster Sensor dazu eingerichtet ist, IR-Strahlung bei einer ersten Wellenlänge von etwa 9,2 µm zu detektieren, und mindestens ein anderer erster Sensor dazu eingerichtet ist, IR-Strahlung bei einem zweiten Wellenlängenbereich zu detektieren, welcher die erste Wellenlänge umfasst und ferner eine andere Wellenlänge oder einen anderen Wellenlängenbereich umfasst, und
(c) Analysieren der detektierten IR-Strahlung für die qualitative und/oder quantitative Bestimmung von Glukose,
wobei die Strahlungsquelle (a), die Erfassungseinheit (b) und die Analyseeinheit (c) innerhalb eines Gehäuses integriert sind und
wobei das Gehäuse eine erste Fläche umfassend einen Bildschirm umfasst, wobei der Bildschirm mindestens teilweise aus einem Material hergestellt ist, welches für von der Strahlungsquelle (a) emittierte NIR/VIS-Strahlung und für von der Erfassungseinheit (b) detektierte IR-Strahlung optisch transparent ist,
wobei gegebenenfalls Schritt (b) ferner (iii) ein Durchführen einer Temperaturmessung umfasst und Schritt (c) gegebenenfalls ferner ein Durchführen einer temperaturkompensierten Analyse der detektierten IR-Strahlung umfasst, und
wobei die Glukosekonzentration quantitativ bestimmt wird und/oder wobei die Änderungsrate der Glukosemenge bestimmt wird, insbesondere nicht quantitativ bestimmt wird.

9. Verfahren nach Anspruch 8,
wobei der mindestens eine andere erste glukosespezifische Sensor der Erfassungseinheit dazu eingerichtet ist, IR-Strahlung bei einer Wellenlänge von etwa 9,2 µm und zusätzlich bei einer Wellenlänge von etwa 9,4 µm und/oder etwa 9,6 µm zu detektieren, insbesondere bei einer Wellenlänge von etwa 9,4 µm und etwa 9,6 µm.

10. Verfahren nach Anspruch 8 oder 9, wobei die Körperflüssigkeit Blut ist.

## Revendications

1. Dispositif comprenant un système non invasif pour déterminer le glucose dans un fluide corporel d'un sujet, dans lequel le dispositif comprend un boîtier (80), dans lequel le dispositif comprend :
(a) une source de rayonnement (82) adaptée pour émettre un rayonnement visible (VIS)/proche infrarouge (NIR) dans la plage d'environ 400 nm à environ 1500 nm dans une partie du corps (86) dudit sujet, dans lequel la partie du corps (86) est particulièrement choisie parmi un bout de doigt, une pluralité de bouts de doigts et une paume, et dans lequel la source de rayonnement (82) est en outre adaptée pour que la partie du corps irradiée (86) absorbe l'énergie électromagnétique, ce qui entraîne une augmentation locale de la température des tissus et une augmentation de l'émission de rayonnement IR dans la plage de longueurs d'onde d'environ 5 µm à environ 12 µm,
(b) une unité de détection (83) pour détecter le rayonnement IR émis par la partie du corps précédemment irradiée dudit sujet dans la plage d'environ 5 µm à environ 12 µm,
dans laquelle ladite unité de détection (83) est adaptée pour (i) détecter le rayonnement IR ayant au moins une longueur d'onde ou une plage de longueurs d'onde où l'intensité du rayonnement IR détecté dépend de la concentration de glucose dans le fluide corporel dudit sujet,
dans lequel l'intensité du rayonnement IR émis diminue avec l'augmentation de la concentration en glucose et l'intensité du rayonnement IR émis augmente avec une diminution de la concentration en glucose,
dans lequel l'unité de détection (83) comprend une pluralité de capteurs spécifiques au glucose, dans lequel un premier capteur est adapté pour détecter un rayonnement IR à une première longueur d'onde d'environ 9,2 µm et au moins un autre premier capteur est adapté pour détecter un rayonnement IR à une deuxième plage de longueurs d'onde qui englobe la première longueur d'onde et englobe en outre une autre longueur d'onde ou plage de longueurs d'onde, et
dans lequel l'unité de détection (83) est adaptée pour (ii) détecter un rayonnement IR ayant au moins une longueur d'onde ou une plage de longueurs d'onde où l'intensité du rayonnement IR détecté est sensiblement indépendante de la concentration de glucose dans le fluide corporel dudit sujet, et
(c) une unité d'analyse pour la détermination qualitative et/ou quantitative du glucose sur la base du rayonnement infrarouge détecté dans l'unité de détection (b), et
dans lequel le boîtier (80) comprend une première face comprenant un écran (81), dans lequel l'écran (81) est au moins partiellement constitué d'un matériau qui est optiquement transparent pour le rayonnement NIR/VIS émis par la source de rayonnement (a) (82) et au rayonnement IR détecté par l'unité de détection (b) (83),
dans lequel la source de rayonnement (a) (82), l'unité de détection (b) (83) et l'unité d'analyse (c) sont incorporées dans le boîtier (80).

2. Dispositif selon la revendication 1,
dans lequel au moins un autre premier capteur spécifique au glucose de l'unité de détection (83) est adapté pour détecter un rayonnement IR à une longueur d'onde d'environ 9,2 µm et en outre à une longueur d'onde d'environ 9,4 µm et/ou d'environ 9,6 µm, en particulier à une longueur d'onde d'environ 9,4 µm et d'environ 9,6 µm.

3. Dispositif selon la revendication 1 ou 2,
dans lequel la source de rayonnement (a) (82) est adaptée pour émettre un rayonnement à travers l'écran (81), et/ou
dans lequel l'unité de détection (b) (83) est adaptée pour détecter le rayonnement entrant dans le boîtier (80) à travers l'écran (81), et/ou
dans lequel le dispositif est un dispositif mobile, et qui est en particulier choisi parmi un téléphone intelligent, une montre intelligente, une tablette et un appareil de suivi de la condition physique,
et/ou
dans lequel le matériau optiquement transparent est choisi parmi un matériau inorganique tel que CaF₂ et/ou BaF₂ et parmi un matériau organique tel qu'un matériau plastique, et/ou
dans lequel le matériau optiquement transparent a une épaisseur d'environ 0,2 mm à environ 2 mm, en particulier d'environ 0,3 mm à environ 1 mm.

4. Le dispositif selon l'une quelconque des revendications 1 à 3, dans lequel le fluide corporel est du sang.

5. Dispositif selon l'une quelconque des revendications 1 à 4, qui comprend en outre un élément de lentille adapté pour focaliser le rayonnement IR provenant de la partie du corps vers l'unité de détection (b), en particulier vers au moins un capteur de l'unité de détection (b),
dans lequel l'élément de lentille est incorporé dans le boîtier (80) et en particulier dans lequel l'élément de lentille comprend une lentille de Fresnel IR ou un réseau comprenant une pluralité de lentilles de Fresnel IR.

6. Le dispositif selon l'une quelconque des revendications 1 à 5, dans lequel l'unité de détection (b) (83) est adaptée pour détecter le rayonnement IR émis par la partie du corps irradiée pendant une période de temps, dans lequel la partie du corps est irradiée par un rayonnement VIS/NIR pendant au moins une partie de ladite période de temps, et/ou
dans lequel l'unité d'analyse (c) est adaptée pour une analyse dépendante du temps du rayonnement IR détecté, dans lequel un signal de mesure est enregistré pendant une période de temps.

7. Utilisation du dispositif selon l'une quelconque des revendications 1 à 6 pour déterminer de manière non invasive le glucose dans un fluide corporel d'un sujet, dans lequel le fluide corporel est du sang, et dans lequel le taux de variation de la quantité de glucose dans le sang est déterminé.

8. Procédé pour déterminer de manière non invasive le glucose dans un fluide corporel d'un sujet, comprenant les étapes suivantes :
(a) irradier une partie du corps dudit sujet avec un rayonnement visible (VIS)/ proche infrarouge (NIR) dans la plage de longueurs d'onde d'environ 400 nm à environ 1500 nm, ou d'environ 500 nm à environ 1500 nm, dans lequel la partie du corps irradiée absorbe l'énergie électromagnétique, ce qui entraîne une augmentation locale de la température des tissus et une augmentation de l'émission de rayonnement IR dans la plage de longueurs d'onde d'environ 5 µm à environ 12 µm,
(b) détecter le rayonnement IR émis par la partie du corps précédemment irradiée dudit sujet dans la plage de longueurs d'onde d'environ 5 µm à environ 12 µm, comprenant séparément (i) la détection du rayonnement IR ayant au moins deux longueurs d'onde ou plages de longueurs d'onde où l'intensité du rayonnement IR détecté dépend de la concentration de glucose dans le fluide corporel dudit sujet, dans lequel l'intensité du rayonnement IR émis diminue avec l'augmentation de la concentration en glucose et l'intensité du rayonnement IR émis augmente avec la diminution de la concentration en glucose, et
(ii) détecter un rayonnement IR ayant au moins une longueur d'onde ou une plage de longueurs d'onde où l'intensité du rayonnement IR détecté est sensiblement indépendante de la concentration en glucose dans le fluide corporel dudit sujet, comprenant la détection du rayonnement IR à l'aide d'une pluralité de capteurs spécifiques au glucose, dans laquelle un premier capteur est adapté pour détecter le rayonnement IR à une première longueur d'onde d'environ 9,2 µm et au moins un autre premier capteur est adapté pour détecter le rayonnement IR à une deuxième plage de longueurs d'onde qui englobe la première longueur d'onde et englobe en outre une autre longueur d'onde ou plage de longueurs d'onde, et
(c) analyser le rayonnement IR détecté pour la détermination qualitative et/ou quantitative du glucose,
dans lequel la source de rayonnement (a), l'unité de détection (b) et l'unité d'analyse (c) sont incorporées dans un boîtier, et
dans lequel le boîtier comprend une première face comprenant un écran, dans lequel l'écran est au moins partiellement constitué d'un matériau qui est optiquement transparent pour le rayonnement NIR/VIS émis par la source de rayonnement (a) et pour le rayonnement IR détecté par l'unité de détection (b), dans lequel, en option, l'étape (b) comprend en outre (iii) la réalisation d'une mesure de température, et l'étape (c) comprend en outre, en option, la réalisation d'une analyse compensée en température du rayonnement IR détecté, et
dans lequel la concentration de glucose est déterminée quantitativement, et/ou dans lequel le taux de variation de la quantité de glucose est déterminé, en particulier de manière non quantitative.

9. Procédé selon la revendication 8, dans lequel au moins un autre premier capteur spécifique au glucose de l'unité de détection est adapté pour détecter un rayonnement IR à une longueur d'onde d'environ 9,2 µm et en outre à une longueur d'onde d'environ 9,4 µm et/ou d'environ 9,6 µm, en particulier à une longueur d'onde d'environ 9,4 µm et d'environ 9,6 µm.

10. Procédé selon la revendication 8 ou 9, dans lequel le fluide corporel est du sang.
